# EUROPEAN PATENT SPECIFICATION

(11) **EP 2 160 462 B1**
(45) Date of publication and mention of the grant of the patent: **26.07.2017**
(21) Application number: 08774359.7
(22) Date of filing: 26.06.2008
(51) Int. Cl.: C12N 15/09, C12N 15/86, C07K 14/11, C12N 7/04

(54) **LINEAR CONSTRUCTS FOR EXPRESSION OF INFLUENZA VIRUS**
LINEARKONSTRUKTE ZUR EXPRESSION VON INFLUENZAVIRUS
CONSTRUCTIONS LINEAIRES POUR L'EXPRESSION DU VIRUS DE LA GRIPPE

(30) Priority: 27.06.2007 US 946651 P; 05.10.2007 EP 07450177
(43) Date of publication of application: 10.03.2010
(73) Proprietor: Nanotherapeutics, Inc., Alachua, FL 32615 (US)
(72) Inventor: WOLSCHEK, Markus, A-1090 Vienna (AT); EGOROV, Andrej, A-1200 Vienna (AT); BERGMANN, Michael, A-3400 Klosterneuburg (AT); MUSTER, Thomas, A-1180 Vienna (AT); KITTEL, Christian, A-1020 Vienna (AT)
(74) Representative: Grund, Martin
(86) International application number: PCT/EP2008/058182
(87) International publication number: WO 2009/000891

(56) References cited:
- WO-A-00/56914
- WO-A-00/60050
- WO-A-01/04333
- WO-A-01/83794
- WO-A-03/091401
- WO-A-2005/062820
- WO-A2-2007/044024
- WO-A2-2008/156681
- US-B1- 6 468 544
- US-B1- 6 669 943
- OZAWA M, GOTO H, HORIMOTO T, KAWAOKA Y.: "An adenovirus vector-mediated reverse genetics system for influenza A virus generation." J VIROL., vol. 81, no. 17, 27 June 2007 (2007-06-27), - September 2007 (2007-09) pages 9556-9559, XP002471230
- HOFFMANN E ET AL: "UNIDIRECTIONAL RNA POLYMERASE I-POLYMERASE II TRANSCRIPTION SYSTEM FOR THE GENERATION OF INFLUENZA A VIRUS FROM EIGHT PLASMIDS" JOURNAL OF GENERAL VIROLOGY, SOCIETY FOR GENERAL MICROBIOLOGY, SPENCERS WOOD, GB, vol. 81, no. PART 12, December 2000 (2000-12), pages 2843-2847, XP000982040 ISSN: 0022-1317
- HOFFMANN E ET AL: "Rescue of influenza B virus from eight plasmids" PROCEEDINGS OF THE NATIONAL ACADEMY OF SCIENCES OF USA, NATIONAL ACADEMY OF SCIENCE, WASHINGTON, DC, US, vol. 99, no. 17, 20 August 2002 (2002-08-20), pages 11411-11416, XP002973258 ISSN: 0027-8424 cited in the application
- NEUMANN G ET AL.: "An improved reverse genetics system for influenza A virus generation and its implications for vaccine production" PNAS, vol. 102, no. 46, 15 November 2005 (2005-11-15), pages 16825-16829, XP002471231
- NEUMANN G ET AL: "Generation of influenza A virus from cloned cDNAs--historical perspective and outlook for the new millenium" REVIEWS IN MEDICAL VIROLOGY, CHICHESTER, GB, vol. 12, no. 1, January 2002 (2002-01), pages 13-30, XP002314285 ISSN: 1052-9276
- "An NLS peptide covalently linked to linear DNA does not enhance transfection efficiency of cationic polymer based gene delivery systems." J GENE MED., vol. 7, no. 2, February 2005 (2005-02), pages 208-217, XP002471232 cited in the application
- RECOMBINATION DURING EARLY HERPES SIMPLEX VIRUS TYPE 1 INFECTION IS MEDIATED BY CELLULAR PROTEINS.: "Yao XD, Elias P." J BIOL CHEM., vol. 276, no. 4, 7 November 2000 (2000-11-07), - 26 January 2001 (2001-01-26) pages 2905-2913, XP002471233 cited in the application
- ERICH HOFFMANN ET AL: "A DNA transfection system for generation of influenza A virus from eight plasmids", PROCEEDINGS OF THE NATIONAL ACADEMY OF SCIENCES - PNAS, NATIONAL ACADEMY OF SCIENCES, US, vol. 97, no. 11, 23 May 2000 (2000-05-23), pages 6108-6113, XP008131838, ISSN: 0027-8424, DOI: 10.1073/PNAS.100133697
- FELGNER ET AL: "debugging expression screening", NATURE BIOTECHNOLOGY, NATURE PUBLISHING GROUP, NEW YORK, NY, US, vol. 17, 1 April 1999 (1999-04-01), pages 329-330, XP002146014, ISSN: 1087-0156, DOI: 10.1038/7880
- A. H. Reid: "The origin of the 1918 pandemic influenza virus: a continuing enigma", Journal of General Virology, vol. 84, no. 9, 1 September 2003 (2003-09-01), pages 2285-2292, XP055153485, ISSN: 0022-1317, DOI: 10.1099/vir.0.19302-0
- HOFFMANN E ET AL: "''Ambisense'' Approach for the Generation of Influenza A Virus: vRNA and mRNA Synthesis from One Template", VIROLOGY, ELSEVIER, AMSTERDAM, NL, vol. d22bis cité PA lettre 18.6.2015267, no. 2, 15 February 2000 (2000-02-15), pages 310-317, XP004436115, ISSN: 0042-6822, DOI: 10.1006/VIRO.1999.0140
- BRATATI SAHA ET AL: "The Adenovirus Genome Contributes to the Structural Stability of the Virion", VIRUSES, vol. 6D24 cité, no. 9, 24 September 2014 (2014-09-24), pages 3563-3583, XP055270616, DOI: 10.3390/v6093563
- BENTIN T ET AL: "ENHANCED PEPTIDE NUCLEIC ACID BINDING TO SUPERCOILED DNA: POSSIBLE IMPLICATIONS FOR DNA BREATHING DYNAMICS", BIOCHEMISTRY, AMERICAN CHEMICAL SOCIETY, US, vol. 35, no. 27, 1 January 1996 (1996-01-01), pages 8863-8869, XP002928575, ISSN: 0006-2960, DOI: 10.1021/BI960436K
- EGHOLM M ET AL: "EFFICIENT PH-INDEPENDENT SEQUENCE-SPECIFIC DNA BINDING BY PSEUDOISOCYTOSINE-CONTAINING BIS-PNA", NUCLEIC ACIDS RESEARCH, INFORMATION RETRIEVAL LTD, GB, vol. 23, no. 2, 25 January 1995 (1995-01-25), pages 217-222, XP002022883, ISSN: 0305-1048
- NIELSEN P E ET AL: "SEQUENCE-SELECTIVE RECOGNITION OF DNA BY STRAND DISPLACEMENT WITH ATHYMINE-SUBSTITUTED POLYAMIDE", SCIENCE, AMERICAN ASSOCIATION FOR THE ADVANCEMENT OF SCIENCE, US, vol. 254, 6 December 1991 (1991-12-06), pages 1497-1500, XP000650416, ISSN: 0036-8075, DOI: 10.1126/SCIENCE.1962210
- Vadim V Demidov ET AL.: "Kinetics and mechanism of polyamide ("peptide") nucleic acid binding to duplex DNA.", Proc Natl Acad Sci U S A., vol. 92 March 1995 (1995-03), pages 2637-2641, XP055272478, Retrieved from the Internet: URL:http://www.ncbi.nlm.nih.gov/pmc/articl es/PMC42273/pdf/pnas01485-0229.pdf [retrieved on 2016-05-12]
- DEMIDOV V V ET AL: "Stability of peptide nucleic acids in human serum and cellular extracts", BIOCHEMICAL PHARMACOLOGY, ELSEVIER, US, vol. 48, no. 6, 15 September 1994 (1994-09-15), pages 1310-1313, XP025552808, ISSN: 0006-2952, DOI: 10.1016/0006-2952(94)90171-6 [retrieved on 1994-09-15]

## Description

The present field of the invention relates to a novel linear expression construct for expressing segments of viral RNA, preferably influenza viruses, free of any amplification and/or selection sequences and comprising an RNA polymerase I (poll) promoter and a poll termination signal, inserted between a RNA polymerase II (polII) promoter and a polyadenylation signal. The disclosure also covers the use of this expression construct for the production of virus particles.

### Background:

Negative-strand RNA viruses are a group of animal viruses that comprise several important human pathogens, including influenza, measles, mumps, rabies, respiratory syncytial, Ebola and hantaviruses.

The genomes of these RNA viruses can be unimolecular or segmented, single stranded of (-) polarity. Two essential requirements are shared between these viruses: the genomic RNAs must be efficiently copied into viral RNA, a form which can be used for incorporation into progeny virus particles and transcribed into mRNA which is translated into viral proteins. Eukaryotic host cells typically do not contain a machinery for replicating RNA templates or for translating polypeptides from a negative stranded RNA template. Therefore negative strand RNA viruses encode and carry an RNA-dependent RNA polymerase to catalyze synthesis of new genomic RNA for assembly into progeny and mRNAs for translation into viral proteins.

Genomic viral RNA must be packaged into viral particles in order for the virus to be transmitted. The process by which progeny viral particles are assembled and the protein/protein interactions occur during assembly are similar within the RNA viruses. The formation of virus particles ensures the efficient transmission of the RNA genome from one host cell to another within a single host or among different host organisms. Virus families containing enveloped single-stranded RNA of the negative-sense genome are classified into groups having non-segmented genomes (Paramyxoviridae, Rhabdoviridae, Filoviridae and Borna Disease Virus, Togaviridae) or those having segmented genomes (Orthomyxoviridae, Bunyaviridae and Arenaviridae). The Orthomyxoviridae family includes the viruses of influenza, types A, B and C viruses, as well as Thogoto and Dhori viruses and infectious salmon anemia virus.

The influenza virions consist of an internal ribonucleoprotein core (a helical nucleocapsid) containing the single-stranded RNA genome, and an outer lipoprotein envelope lined inside by a matrix protein (M1). The segmented genome of influenza A virus consists of eight molecules of linear, negative polarity, single-stranded RNAs which encodes eleven (some influenza A strains ten) polypeptides, including: the RNA-dependent RNA polymerase proteins (PB2, PB1 and PA) and nucleoprotein (NP) which form the nucleocapsid; the matrix membrane proteins (M1 , M2); two surface glycoproteins which project from the lipid containing envelope: hemagglutinin (HA) and neuraminidase (NA); the nonstructural protein (NS1) and nuclear export protein (NEP). Most influenza A strains also encode an eleventh protein (PB1 -F2) believed to have proapoptotic properties.

Transcription and replication of the genome takes place in the nucleus and assembly occurs via budding on the plasma membrane. The viruses can reassort genes during mixed infections. Influenza virus adsorbs via HA to sialyloligosaccharides in cell membrane glycoproteins and glycolipids. Following endocytosis of the virion, a conformational change in the HA molecule occurs within the cellular endosome which facilitates membrane fusion, thus triggering uncoating. The nucleocapsid migrates to the nucleus where viral mRNA is transcribed. Viral mRNA is transcribed by a unique mechanism in which viral endonuclease cleaves the capped 5'- terminus from cellular heterologous mRNAs which then serve as primers for transcription of viral RNA templates by the viral transcriptase. Transcripts terminate at sites 15 to 22 bases from the ends of their templates, where oligo(U) sequences act as signals for the addition of poly(A) tracts. Of the eight viral RNA molecules so produced, six are monocistronic messages that are translated directly into the proteins representing HA, NA, NP and the viral polymerase proteins, PB2, PB1 and PA. The other two transcripts undergo splicing, each yielding two mRNAs which are translated in different reading frames to produce M1, M2, NS1 and NEP. In other words, the eight viral RNA segments code for eleven proteins: nine structural and 2 nonstructural (NS1 and the recently identified PB1-F2) proteins.

The generation of modern vaccines for influenza viruses especially for highly pathogenic avian influenza viruses relies on the use of reverse genetics which allows the production of influenza viruses from DNA. The first development of a reverse genetic system for construction of negative-strand RNA influenza viruses involved the transfection of a single viral gene mixed with in-vitro reconstituted ribonucleoprotein (RNP) complexes and subsequent infection with an influenza helper virus. RNP complexes were made by incubating synthetic RNA transcripts with purified NP and polymerase proteins (PB1, PB2 and PA) from influenza viruses, the helper virus was used as an intracellular source of viral proteins and of the other vRNAs (Luytjes et al., 1989, Cell, 59, 1107-1113). Neumann et al. (1994, Virology, 202, 477-479) achieved RNP formation of viral model RNAs in influenza-infected cells after expression of RNA from a murine RNA polymerase I promoter-responsive plasmid.

Pleschka et al. (1996, J. Virol., 4188-4192) described a method wherein RNP complexes were reconstituted from plasmid-based expression vectors. Expression of a viral RNA- like transcript was achieved from a plasmid containing a truncated human polymerase I (poll) promoter and a ribozyme sequence that generated a 3' end by autocatalytic cleavage. The poll-driven plasmid was cotransfected into human 293 cells with poll I- responsive plasmids that express the viral PB1, PB2, PA and NP proteins. Yet, transfection efficiency was very low, approx. 10 transfectant virus particles per transfection. Additionally, this plasmid-based strategy was dependent on the aid of a helper virus.

In WO 01/04333 segmented negative-strand RNA viruses were constructed using a set of 12 expression plasmids for expressing genomic vRNA segments and RNP proteins. The vectors described in WO 01/04333 were based on well known pUC19 or pUC18 plasmids. According to the description this system requires a set of 8 plasmids expressing all 8 segments of influenza virus together with an additional set of 4 plasmids expressing nucleoprotein and subunits of RNA-dependent RNA polymerase (PB1, PB2, PA and NP).

WO 00/60050 covers a set of at least two vectors comprising a promoter operably linked to an influenza virus segment cDNA (PA, PB1, PB2, HA, NP, NA, M) linked to a transcription termination sequence and at least two vectors comprising a promoter operably linked to an influenza virus segment DNA (PA, PB1 , PB2, NP). The use of a large number of different vectors was tried to overcome by using plasmids with eight RNA polymerase I transcription cassettes for viral RNA synthesis combined on one plasmid.

WO 01/83794 discloses circular expression plasmids comprising an RNA polymerase I (polI) promoter and a poll termination signal, inserted between a RNA polymerase II (polII) promoter and a polyadenylation signal. The term vector according to this application is described as a plasmid which generally is a self-contained molecule of double-stranded DNA that can accept additional foreign DNA and which can be readily introduced into a suitable host cell.

Ozawa et al. (J. Virol. 2007, 81(17):9556-9559) describe a bidirectional adenoviral cDNA construct comprising an RNA polymerase I (polI) promoter and a poll termination signal inserted between an RNA polymerase II (polII) promoter and a polyadenylation signal, free of any amplification and/or selection sequences, for expression of influenza A viral segments and production of recombinant influenza particles. Similar constructs are described in WO 2008/156681.

Epidemics and pandemics caused by viral diseases are still claiming human lives and are impacting global economy. Influenza is responsible for millions of lost work days and visits to the doctor, hundreds of thousands of hospitalizations worldwide (Couch 1993, Ann. NY. Acad. Sci 685;803,), tens of thousands of excess deaths (Collins & Lehmann 1953 Public Health Monographs 213:1 ; Glezen 1982 AmJ. Public Health 77:712) and billions of Euros in terms of health-care costs (Williams et al. 1988, Ann. Intern. Med. 108:616). When healthy adults get immunized, currently available vaccines prevent clinical disease in 70-90% of cases. This level is reduced to 30-70% in those over the age of 65 and drops still further in those over 65 living in nursing homes (Strategic Perspective 2001 : The Antiviral Market. Datamonitor. p. 59). The virus's frequent antigenic changes further contribute to a large death toll because not even annual vaccination can guarantee protection. Hence, the U.S. death toll rose from 16,363 people in 1976/77 to four times as many deaths in 1998/99 (Wall Street Journal, Flu- related deaths in US despite vaccine researches. January 7, 2003).

Especially in case of the outbreak of pandemic viral diseases, it can be of utmost importance to provide vaccinations or treatments immediately after outbreak of the disease.

In view of the urgent need for providing efficient protection and treatment of viral diseases there is a still high demand for the development of economic, fast and efficient expression systems for virus production which can overcome the disadvantages and difficulties of the present expression systems.

### Brief description of the invention

The invention is defined by the appended claims.

The inventors have surprisingly shown that the use of a linear expression construct free of any conventionally plasmid-based bacterial amplification and/or selection sequences comprising a viral gene cloned into a cassette of an RNA polymerase I (polI) promoter and a poll termination signal, inserted between a RNA polymerase II (polII) promoter and a polyadenylation signal provides a highly economic and efficient tool for fast rescue of viral particles. In contrast to the plasmids used by known technologies, no cloning steps in bacterial cells are needed. Therefore, the time needed for transfection and expression of viral particles can be highly reduced, preferably from at least several weeks to few days.

For example, the linear expression construct according to the invention can be used for developing vaccines against RNA viruses, specifically against influenza viruses either of wildtype, mutant or reassortant strains. This provides a tool for fast generation of any virus vaccine needed in case of occurrence of influenza epidemics or pandemics.

Circularization of the linear expression construct using short linker sequences is also disclosed. Also methods can be provided wherein the linear expression constructs are used for the production of viral particles, or, alternatively, wherein some of the viral gene segments of a complete virus can be expressed via a circularized expression construct and at least one of the gene segments is expressed via a linearized expression construct according to the present invention.

### Figures

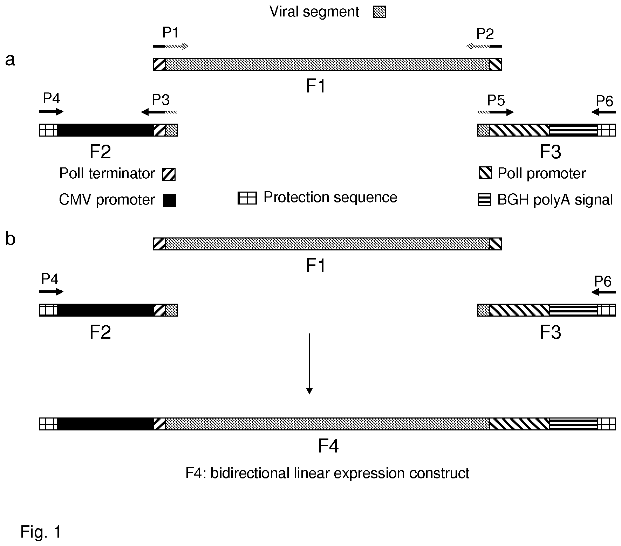

Figure 1 shows a schematic diagram of the generation of linear bidirectional expression constructs, a) Fragments F1, F2 and F3 are generated separately by PCR amplification. b) Fragment F4 is generated by overlapping PCR using the oligonucleotides P4 and P6.

### Detailed description of the invention

The invention in its broadest sense is as defined in the independent claims.

As already discussed in the introduction the currently used reverse genetics rescue system of segmented RNA viruses still requires the transfection of a high number of different plasmids and/or still suffers from the need for subcloning of viral genes and subsequent amplification in bacterial cells to develop a sufficient amount of plasmids. Plasmid DNA has to be sequenced, purified from bacteria for each individual clone and can only then be further used for transfection of animal cells. This method is time consuming, costly and difficult to automate.

The present invention now provides a linear expression cassette free of any conventionally plasmid-based bacterial amplification and/or selection sequences comprising a viral gene cloned into a cassette of an RNA polymerase I (polI) promoter and a poll termination signal, inserted between an RNA polymerase II (polII) promoter and a polyadenylation signal which can be used for expressing virus particles.

A "cassette" refers to a DNA coding sequence or segment that codes for an expression product that can be inserted into the expression construct at defined restriction sites. The cassette restriction sites are developed to ensure the insertion of the cassette in the correct reading frame.

The inventive constructs allow the transcription of the cDNA into mRNA and a full length negative stranded (sense) vRNA (bidirectional transcription) or mRNA and full length positive stranded (sense) cRNA (unidirectional transcription).

For unidirectional transcription, the gene is located downstream of the poll promoter and polII promoter. The polII promoter produces capped positive sense viral mRNA and the poll promoter produces uncapped positive-sense viral cRNA. For bidirectional transcription, the gene is located between the upstream polII promoter and the downstream poll promoter. The polII promoter transcription produces capped positive sense viral mRNA and the poll promoter transcription produces uncapped negative-sense vRNA.

The inventors surprisingly showed that such linear expression construct can be efficiently used for transfection of cells and expression of complete viral particles although it was often described in the art that transfection of animal cells with linear fragments can lead to fast decomposition of these fragments due to cellular exonuclease degradation (van der Aa et al. 2005, J Gene Med. 7:208-17) or result in increased apoptosis of the transfected cells (Yao et al. 2001, J Biol Chem. 276:2905-13).

The linear expression constructs do not contain any selection or amplification sequences that are needed for amplification of plasmids in bacterial cells. Neither ori (origin of replication)-sequences nor antibiotics resistance genes or any other selection markers are contained. The linear expression construct comprises additional protection sequences at the N- and/or C-terminus of the construct. These protection sequences can be peptide nucleic acid sequences (PNAs) as described in WO 00/56914.

These PNAs are nucleic acid analogs in which the entire deoxyribose-phosphate backbone has been exchanged with a chemically completely different, but structurally homologous, polyamide (peptide) backbone containing 2-aminoethyl glycine units. PNA "clamps" have also been shown to increase stability, wherein two identical PNA sequences are joined by a flexible hairpin linker containing three 8-amino-3,6-dioxaoctanoic acid units. When a PNA is mixed with a complementary homopurine or homopyrimidine DNA target sequence (clamp binding sequence), a PNA-DNA-PNA triplex hybrid can form which is extremely stable (Bentin et al., 1996, Biochemistry, 35, 8863-8869, Egholm et al., 1995, Nucleic Acids Res., 23, 217-222, Nielsen et al., Science, 1991, 254, 1497-1500, Demidov et al., Proc. Natl. Acad. Sci., 1995, 92, 2637-2641). They have been shown to be resistant to nuclease and protease digestion (Demidov et al., Biochem.Pharm., 1994, 48, 1010-1013). The constructs of the invention comprise a homopurine or homopyrimidine PNA clamp binding sequence and optionally a PNA clamp sequence comprising two PNA clamp half-sites joined by a flexible hairpin linker containing three 8-amino-3,6-dioxaoctanoic acid units. In view of protection against cellular nucleases, protection sequences are also disclosed that can be any nucleic acid sequences of a length of at least 20 nucleic acids, preferably at least 50 nucleic acids, more preferably at least 100 nucleic acids. These nucleic acids can be digested by nucleases thereby protecting or delaying degradation of the core sequences, i.e promoter sequences, viral sequences and termination sequences of the expression construct.

The linear expression construct of the invention comprises at least one Influenza virus gene segment inserted between the poll promoter and the termination signal.

The term "viral gene" as used in the present invention means a DNA or a cDNA sequence or RT-PCR amplification product coding for or corresponding to a particular sequence of amino acids. The term gene also includes full length genes and fragments thereof as well as derivatives comprising modifications of the natural gene sequence. These modifications can be deletions, substitutions or insertions of nucleotides resulting in amino acid modifications as well as silent mutations wherein change of one or more nucleotides do not result in alterations of the amino acid encoded at that position. Modifications can also include functional conservative mutants wherein the change of a given amino acid residue does not lead to an alteration of the overall confirmation and function of the polypeptide.

It includes various mutants, sequence conservative variants and functionally conservative RNA virus gene segments, preferably negative strand RNA virus gene segments.

Modifications can be introduced by random mutagenesis techniques or by site-directed mutagenesis, e.g. PCR-based sequence modifications. Modification of one or more individual gene segments of an RNA virus can permit development of attenuated or replication deficient viruses.

According to the invention the term "replication deficient" is defined as replication rate in interferon competent host cells that is at least less than 5%, preferably less than 1 %, preferably less than 0.1 % than wild type influenza virus as determined by hemagglutination assay, TCID50 assay or plaque assay as well known in the art.

The inventive expression construct is used for the expression of influenza genomes. For example, a viral cDNA corresponding to each gene in the target virus genome is inserted into a linear expression construct of the invention resulting in a set of linear expression constructs covering the complete viral genome.

To amplify these constructs, PCR technology as known in the art can be used, avoiding time-consuming cloning, amplification, sequencing and purification in bacterial host cells. According to a preferred embodiment the viral segments are derived from influenza, types A, B and C viruses.

The viral gene segment can be selected from the group consisting of a PA, PB1, PB2, HA, NA, NP, M or NS gene or part thereof. Alternatively, the viral NS gene segment can be coding for a non-functional NS1 protein. This can be any modification within the NS gene, i.e. a substitution, insertion or deletion of nucleic acids. Preferably the modifications of the NS gene diminish or eliminate the ability of the NS gene product to antagonize the cellular IFN response. Examples for influenza viruses having reduced or no interferon antagonist activity are described in detail in US 6,669,943 or US 6,468,544.

In a preferred embodiment, reassortant viruses can be provided, wherein each viral segment can be selected from a specific virus strain, for example H1N1, H3N2, even H5N1 or any seasonal strain that is identified to be most relevant in causing influenza. The reassortant viruses can thereby carry the desired antigenic characteristics in a background that allows efficient production in a host cell.

For example, the reassorted influenza viruses combine the genes for the surface glycoproteins hemagglutinin (HA) and/or neuraminidase (NA) of actual interpandemic viruses with five or six or seven RNA segments coding for other proteins from the attenuated master strain (6/2 combination) or 7/1 reassortants or 5/3 reassortants containing M genes of different origin respectively.

These reassortment viruses can then be used as virus seed for the production of virions to produce vaccines.

The term "virions" refer to viral particles, which when first produced are fully infectious in the host cell, from host cells transfected or co-transfected with an expression system of the invention. This system then produces vRNA and viral proteins (from viral RNA translation), thereby resulting in the assembly of infectious virus particles.

The linear expression constructs of the invention can also be combined into a set of at least two expression constructs. For example, a set of eight linear expression constructs each containing one viral gene segment of PA, PB1, PB2, HA, NA, NP, M and NS or part thereof of influenza virus can be provided.

Alternatively, the linear expression construct can be circularized by peptide linkers and/or overlapping sequences. Various different systems for circularization might be possible like using a 5' oligo with a GGGG extension and a 3' oligo with a CCCC sequence.

Alternatively, after T4 DNA polymerase treatment in presence of dATP and dTTP (to generate sticky ends) could linear constructs be circularized via ligation.

The present invention also provides host cells comprising at least one inventive linear expression construct.

Within the scope of the invention, the term "cells" or "host cells" means the cultivation of individual cells, tissues, organs, insect cells, avian cells, mammalian cells, hybridoma cells, primary cells, continuous cell lines, and/or genetically engineered cells, such as recombinant cells expressing a virus. These can be for example BSC-1 cells, LLC-MK cells, CV-1 cells, CHO cells, COS cells, murine cells, human cells, HeLa cells, 293 cells, Vera cells, MDBK cells, MDCK cells, MDOK cells, CRFK cells, RAF cells, TCMK cells, LLC-PK cells, PK15 cells, WI-38 cells, MRC-5 cells, T-FLY cells, BHK cells, SP2/0 cells, NSO, PerC6 (human retina cells), chicken embryo cells or derivatives, embryonated egg cells, embryonated chicken eggs or derivatives thereof. Preferably the cell line is a Vero cell line.

Besides well known methods to introduce cDNA sequences into expression systems, the present disclosure also provides a further method for easily constructing the linear expression constructs, wherein the viral segments are provided with complementary sequences overlapping with the poll promoter and poll terminator sequences. In that case, three different fragments are combined, annealed and amplified by PCR.

Using the linear expression construct according to the present invention the transformation and amplification of plasmids in bacterial cells is not required. The linear fragments each containing at least one segment of the influenza virus genome or part thereof can be used directly to transfect host cells. The use of these linear constructs provide a system for transfection and expression of virus particles using the construct according to the invention wherein only few days are needed to receive a complete virus particle.

In summary, starting from an influenza virus isolate, transfections can be performed within several hours up to 2-3 days after receiving the virus. In contrast, cloning of the plasmids according to the state of the art would require at least 4-5 days without sequencing. Transfection could be performed on day 5. However, several bacterial clones for each "new" segment have to be tested to optimise the chance of using a correct plasmid. If sequences are available for the respective virus isolate sequencing of several clones for each segment will further delay the process about one day. Thus, transfection could be done on day 6 at the earliest. Complete sequencing without prior sequence information would add 2-3 days for oligonucleotide synthesis According to a further embodiment the invention covers a method of producing the linear expression construct wherein a first DNA fragment containing an Influenza virus segment and a sequence homologous to poll promoter and a sequence homologous to pol I terminator, a second DNA fragment containing a protection sequence, a pol I promoter sequence, a poly A signal sequence and an overlapping sequence of at least 12 nucleotides, complementary to the viral segment and a third DNA fragment containing a protection sequence, a CMV promoter, a pol I terminator sequence, and an overlapping sequence of at least 12 nucleotides complementary to the viral segment are fused together via overlapping PCR and purified by standard purification methods.

According to an alternative method of the disclosure, the DNA fragment containing a viral segment and a sequence homologous to poll promoter and a sequence homologous to pol I terminator can additionally contain at least 5 nucleotides which are introduced to the fragment to serve as complementary sequences for the two fragments comprising the Poll terminator and CMV promoter as well as the Poll promoter and poly A signal which are fused together via PCR techniques.

Both oligonucleotides used for amplification of viral segments can be designed to contain sequences complementary to the DNA fragment comprising the CMV promoter and the Poll terminator and the DNA fragment comprising the Poll promoter and the poly A signal (schematically shown as F2, F3, see Figure 1). Thereby, the length of the overlapping regions between the virus gene segment (schematically shown as F1 in figure 1 ) and the DNA fragment comprising the CMV promoter and the Poll terminator as well as the virus gene segment and DNA fragment comprising the Poll promoter and the poly A signal is increased which should facilitate the second PCR step in which the virus gene segment is fused to the DNA fragment comprising the CMV promoter and the Poll terminator and the DNA fragment comprising the Poll promoter and the poly A signal.

The disclosure also covers the method for producing a negative strand virus particle comprising culturing a host cell under conditions that permit production of viral proteins and vRNA or cRNA. For example, the linear constructs containing all viral gene segments are used to transfect host cells.

Cells are then maintained in culture medium and viral particles can be isolated and purified from the culture supernatant. Optionally, a second purification step can be included to increase purity of the expression construct and to decrease toxicity when transfected into the host cells.

Commercial PCR cleaning kits usually rely on binding of DNA to silica membranes under high-salt conditions. Surprisingly, the investors found that PCR fragments purified via a commercial purification kit via a single purification step can exhibit high toxicity when transfected into Vero cells which can prevent virus rescue.

If PCR products are purified in a second purification step that relies on an anion-exchange resin, purity of PCR products can be increased and to permit virus rescue with increased efficacy.

Alternatively, a single purification step based on anion exchange resins can be performed.

According to a specific aspect of the disclosure, a mixture of Taq polymerase and a proofreading polymerase (e.g. Pfu polymerase) can be used for all PCR amplification steps. Thereby, PCR derived mutations are minimised. Furthermore, the need to incorporate an additional thymidine base into the DNA fragment comprising the CMV promoter and the Poll terminator as well as the virus gene segment and DNA fragment comprising the Poll promoter and the poly A signal is obviated. The disclosure covers a method for generating influenza virus particles wherein at least one linear expression construct is directly transfected into animal host cells, and wherein said host cells are cultured under conditions that influenza virus is expressed and virus particles are collected and purified.

Further, a method is also disclosed, wherein virus particles are incorporated optionally after attenuating or inactivating, into a pharmaceutical composition together with a pharmaceutically acceptable carrier and/or adjuvant as therapeutic or prophylactic medicament. Preferably, the virus particles are used for the production of a pharmaceutical preparation for therapeutic or prophlyactic treatment of infectious diseases, esp. a vaccine formulation. Methods of introduction include but are not limited to intradermal, intramuscular, intraperitoneal, intravenous, intranasal, epidural or oral routes. Introduction by intranasal routes is preferred. It may be desirable to introduce the pharmaceutical preparation into the lungs by any suitable route. Pulmonary administration can also be employed, using e.g. an inhaler or nebulizer or formulate it with an aerosolizing agent. The pharmaceutical preparation can also be delivered by a controlled release system, like a pump.

The medicament can comprise a therapeutically effective amount of the replication deficient virus and a pharmaceutically acceptable carrier. "Pharmaceutically acceptable" means approved by regulatory authorities like FDA or EMEA. The term "carrier" refers to a diluent, adjuvant, excipient or vehicle with which the preparation is administered. Saline solutions, dextrose and glycerol solutions as liquid carriers or excipients like glucose, lactose, sucrose or any other excipients as known in the art to be useful for pharmaceutical preparations can be used. Additionally, also stabilizing agents can be included to increase shelf live of the medicament. Preferably, a ready-to-use infusion solution is provided. Alternatively, the preparation can be formulated as powder which is solved in appropriate aqueous solutions immediately before application.

The amount of the pharmaceutical preparation which will be effective in the treatment of a particular disorder or condition will depend on the nature of the disorder or condition, and can be determined by standard clinical techniques. In addition, in vitro assays may optionally be employed to help identify optimal dosage ranges. The precise dose to be employed in the formulation will also depend on the route of administration, and the seriousness of the disease or disorder, and should be decided according to the judgment of the practitioner and each patient's circumstances. However, suitable dosage ranges for administration are generally up to 8 logs (10⁴ - 5x10⁶ pfu) of replication deficient viruses and can be administered once, or multiple times with intervals as often as needed.

Pharmaceutical preparations comprising 10⁴ -5x10⁶ pfu of replication deficient, attenuated viruses can be administered intranasally, intratracheally, intramuscularly or subcutaneously Effective doses may be extrapolated from dose-response curves derived from in vitro or animal model test systems.

The term "vaccine" is, according to the disclosure, a preparation that can elicit protective immunity to an RNA virus when administered to the subject. It refers to a preparation containing virus, inactivated virus, attenuated virus, split virus or viral protein, like a surface antigen, that can be used to induce protective immunity in a subject. The vaccines are applied at a protective dosage, which is the amount of vaccine, either alone or in combination with one or more adjuvants known to increase immunogenicity, that is sufficient to result in protective immune response.

The foregoing description will be more fully understood with reference to the following examples. Examples not falling within the scope of the claims are for illustrative purposes only.

### Examples

### Example 1 : Generation of a linear H3N2 HA expression construct

The HA segment of a Vero cell culture-derived influenza A H3N2 virus was PCR amplified using the oligonucleotides P1 and P2 (F1 in figure 1 a). Subsequently, two DNA fragments (F2 and F3 in figure 1) derived from pHW2000 (Hoffmann et al. 2000, Proc Natl Acad Sci USA. 97:6108-13) were fused to the HA PCR product by means of overlapping PCR (see figure 1 b). The first DNA fragment (F2) comprises the CMV promoter and the Poll terminator, the second one (F3) comprises the human Poll promoter and the BGH polyA signal. To facilitate generation of the overlapping PCR products, oligonucleotides used for HA amplification were extended on their 5' ends in that P1 contains a sequence complementary to the Poll terminator and P2 contains a sequence complementary to the Poll promoter (see figure 1 a). Similarly, the primers P3 and P5 used for generation of the fragments F1 and F2 were extended on their 5' termini to contain sequences complementary to the 5' and 3' end of the HA (see figure 1 a). Fragments F2 and F3 contain protection sequences derived from sequence described in the pHW2000 backbone. These sequences are not directly involved in transcription of mRNA and vRNA but reduce degradation of the bidirectional expression cassette by exonucleases.

Viral RNA was extracted from a Vero cell culture-derived influenza A H3N2 virus using a Qiagen ViralAmp kit and reverse transcribed using the Uni12 oligonucleotide as described previously (Hoffmann et al. 2001, Arch Virol. 146:2275-89).

The HA segment was amplified with the oligonucleotides shown in the table 1 using a mixture of Pfu Turbo DNA polymerase and Taq DNA polymerase:

**Table 1:**

| | |
|---|---|
| P1 | 5'-CGAAGTTGGGGGGG***AGCAAAAGCAGGGGATAATTCTATTAAC***-3' (SEQ ID No. 1) |
| P2 | 5*'*-GCCGCCGGGTTATT***AGTAGAAACAAGGGTGTTTTTAATTAATGC***-3' (SEQ ID No. 2) |

Nucleotides corresponding to the H3 sequence are shown in italic bold letters, nucleotides homologous to the Poll terminator (P1) and the Poll promoter (P2) are shown in standard capital letters.

The HA F4 PCR product was purified using a Qiaquick PCR Purification kit (Qiagen). PCR fragments F2 and F3 were amplified from pHW2000 plasmid DNA with the primer pairs P3+P4 and P5+P6 (see table 2 and figure 1 a), respectively using a mixture of Pfu Turbo DNA polymerase and Taq DNA polymerase. PCR products F2 and F3 were purified using a QIAquick PCR Purification kit (Qiagen)

**Table 2:**

| | |
|---|---|
| P3 | 5'-***CCTGCTTTTGCT***CCCCCCCAACTTCGGAGGTC-3' (SEQ ID No. 3) |
| P4 | 5'-GGGGTATCAGGGTTATTGTCTCATGAGCGGATAC-3' (SEQ ID No. 4) |
| P5 | 5'-***CCTTGTTTCTACT***AATAACCCGGCGGCCCAAAATGC-3' (SEQ ID No. 5) |
| P6 | 5'-CCCCTTGGCCGATTCATTAATGCAGCTGGTTC3' (SEQ ID No. 6) |

For P3 and P5 nucleotides corresponding to the H3 sequence are shown in italic bold letters, nucleotides complementary to pHW2000 are shown in standard capital letters. For P4 and P6 all nucleotides except the four nucleotides at the 5' ends correspond to pHW2000.

For generation of the full length PCR product (F4) containing the HA, the CMV promoter, the Poll terminator, the Poll promoter and the BGH polyA signal, fragments F1, F2 and F3 were combined and amplified by overlapping PCR with the primers P4 and P6 using a mixture of Pfu Turbo DNA polymerase and Taq DNA polymerase.

Figure 1 shows a schematic diagram of the generation of linear bidirectional expression constructs.

Figure 1a) schematically discloses Fragments F1, F2 and F3 generated separately by PCR amplification.

Fragment F1 contains the respective viral segment and contains extensions complementary to the Poll promoter and Poll terminator. Fragment F2 contains the CMV promoter and the Poll terminator as well as an extension complementary to the respective viral segment. Fragment F3 contains the Poll promoter and the BGH poly adenylation signal as well as an extension complementary to the respective viral segment. Oligonucleotides P1 and P2 used for PCR amplification of F1 fragments are complementary to the respective viral segment. P1 contains a 5' extension complementary to the Poll terminator, P2 contains a 5'extension complementary to the Poll promoter.

Oligonucleotides P3 and P4 are used for PCR amplification of F2 fragments with P3 containing a 5'extension complementary to the respective viral segment.

Oligonucleotides P5 and P6 are used for PCR amplification of F3 fragment with P5 containing a 5'extension complementary to the respective viral segment.

Protection sequences are derived from the pHW2000 backbone and do not contain sequences directly involved in mRNA or vRNA transcription.

### Example 2: Influenza A virus rescue using a linear HA expression construct

Six influenza A H3N2 virus isolates were grown on MDCK cells. The HA segments were PCR amplified (F1 in figure 1) and purified via agarose gel electrophoresis using a Qiaex II kit (Qiagen).

Fragments F2 and F3 were fused to F1 as described in example 1 to yield the full length expression constructs F4. Following purification via agarose gel electrophoresis fragments F4 were PCR reamplified to yield sufficient amounts of DNA for transfection. Finally, the F4 HA DNA fragments were used together with a set of seven plasmids (pHW2000 derivatives) that contain the remaining segments of a Vero adapted Influenza A H1 N1 delNS1 strain (GHB01) for virus rescue on Vero cells.

Figure 1 b discloses fragment F4 generated by overlapping PCR using the oligonucleotides P4 and P6.

Generation of F4 HA DNA fragments was done similarly to the procedure described in example 1. A total amount of 10-20µg F4 HA DNA for each viral isolate were first purified using a Qiaquick kit (Qiagen) and subsequently via a Qiagen Endofree Plasmid kit.

When PCR products were purified in a single step only using a PCR purification kit (Qiaquick, Qiagen) high toxicity was observed upon transfection into Vero cells which prevented virus recue.

Vero cells were maintained in DMEM/F12 medium containing 10% foetal calf serum and 1% Glutamax-I supplement at 37°C.

For virus generation seven derivatives from the published sequence pHW2000 (Hoffmann et al., see above) were generated containing the segments PA, PB1, PB2, NA, M, NP and delNS1 derived from GHB01 as well as a protein expression plasmid coding for influenza A PR8 NS1 (pCAGGS-NS1 (SAM); (Salvatore et al. 2002, J Virol. 76:1206-12)) were used together with the respective F4 HA DNA fragment for cotransfection of Vero cells. Following transfection, to support virus replication, Vero cells were cultured in serum-free medium (Opti-Pro; Invitrogen) in the presence of 5µg/ml trypsin. Three to four days after transfection 50-100% CPE was observed and rescued viruses were frozen or further amplified on Vero cells.

Thus virus rescue for influenza A with one bidirectional expression plasmid replaced by a linear PCR product is feasible.

### Example 3: Influenza A virus rescue entirely from linear expression constructs

Eight linear expression constructs (F4) for a Vero cell-adapted influenza A H1 N1 delNS1 virus (GHB01) were generated by PCR amplification. Eight pHW2000 derivatives that contain the segments of GHB01 served as templates for PCR.

Sufficient amounts of F4 fragments were generated for each segment and subsequently used for virus rescue on Vero cells.

F4 DNA fragment generation was done for each of the eight segments by direct PCR amplification of each whole bidirectional expression cassette containing the respective influenza segment using the respective pHW2000 derivative as template. PCR amplification was performed with oligonucleotides P4 and P6 (shown in the table 3) using a mixture of Pfu DNA Turbo polymerase and Taq DNA polymerase.

**Table 3:**

| | |
|---|---|
| P4 | 5'-GGGGTATCAGGGTTATTGTCTCATGAGCGGATAC-3' (SEQ ID No. 4) |
| P6 | 5'-CCCCTTGGCCGATTCATTAATGCAGCTGGTTC3' (SEQ ID No. 6) |

Sufficient amounts of F4 PCR product (10-20µ) were generated for each segment and purified first using a Qiaquick kit (Qiagen) and subsequently via a Qiagen Endofree Plasmid kit.

Vero cells were transfected with equal amounts of the eight F4 DNA fragments and the NS1 expression plasmid pCAGGS-NS1 (SAM). Following transfection, to support virus replication, Vero cells were cultured in serum-free medium (Opti-Pro; Invitrogen) in the presence of 5µg/ml trypsin.

Complete CPE was observed four days after transfection. Thus, virus rescue from only linear bidirectional influenza A expression constructs is feasible.

In contrast to WO 00/56914 both oligonucleotides used for amplification of viral segments were designed to contain regions complementary to F2 and F3. Thereby, the length of the overlapping regions between F1 and F2 as well as F1 and F3 is increased which should facilitate the second PCR step in which F1 is fused to F2 and F3.

Commercial PCR cleaning kits (e.g. Qiagen) usually rely on binding of DNA to silica membranes under high-salt conditions. PCR fragments purified via a Qiagen PCR purification kit exhibit a high toxicity when transfected into Vero cells which prevent virus rescue.

If PCR products are purified in a second purification step via a Qiagen plasmid purification kit (e.g. Qiagen Endofree plasmid kit) that relies on an anion-exchange resin, PCR products are found to be sufficiently pure to permit virus rescue.

In contrast to WO00/56914 in the present invention a mixture of Taq polymerase and a proofreading polymerase (e.g. Pfu polymerase) is used for all PCR amplification steps. Thereby, PCR derived mutations are minimised. Furthermore, the need to incorporate an additional thymidine base into F2 and F3 (F1 and F2 described in WO00/56914, Fig 2, paragraph 0021 und 0022) is avoided.
## Claims

1. A linear expression construct free of any conventional plasmid-based bacterial amplification and/or selection sequences comprising:
an RNA polymerase I (polI) promoter and a poll termination signal, inserted between a RNA polymerase II (polII) promoter and a polyadenylation signal;
at the N- and/or C-terminus of the construct,
a homopurine or homopyrimidine peptide nucleic acid (PNA) clamp binding sequence that forms a stable PNA-DNA-PNA triplex hybrid with two identical PNA clamp half-sites complementary thereto, and optionally a PNA clamp sequence comprising said two PNA clamp half-sites joined by a flexible hairpin linker containing three 8-amino-3,6-dioxaoctanoic acid units,
wherein a PNA is a nucleic acid analog in which the deoxyribose-phosphate backbone has been exchanged with a polyamide backbone containing 2-aminoethyl glycine units, and wherein a PNA clamp includes two identical PNA sequences joined by a flexible hairpin linker; and
at least one influenza virus gene segment inserted between the poll promoter and the poll termination signal.

2. The linear expression construct according to claim 1 wherein the viral segment is from influenza virus of type A, B or C.

3. The linear expression construct according to claim 1 wherein the viral gene segment is selected from the group consisting of an PA, PB1, PB2, HA, NA, NP, M, NS gene segment or part thereof of influenza virus.

4. The linear expression construct according to any one of claims 1 to 3 wherein the viral NS gene segment is expressing a non-functional NS1 protein

5. The linear expression construct according to any one of claims 1 to 4 wherein the viral gene segment is a cDNA copy or RT-PCR amplification product of said segment.

6. A set of linear expression constructs containing at least two expression constructs according to claims 1 to 5.

7. A set of eight linear expression constructs according to claim 6 each containing at least one viral gene segment of PA, PB1, PB2, HA, NA, NP, M and NS or part thereof of influenza virus.

8. Host cell comprising at least one linear expression construct according to any one of claims 1 to 7.

9. A method of producing a linear expression construct according to any one of claims 1 to 5 wherein:
a first DNA fragment, containing an Influenza virus gene segment inserted between a sequence homologous to pol I promoter and a sequence homologous to pol I terminator,
a second DNA fragment, containing in the following order a protection sequence, a poly A signal sequence, a pol I promoter sequence, and an overlapping sequence of at least 12 nucleotides complementary to the viral segment and
a third DNA fragment, containing in the following order a protection sequence, a CMV promoter, a pol I terminator sequence, and an overlapping sequence of at least 12 nucleotides complementary to the viral segment are fused together via overlapping PCR and purified.

10. A method according to claim 9 comprising at least two purification steps.

## Patentansprüche

1. Ein lineares Expressionskonstrukt, das frei von jeglichen konventionellen Plasmidbasierten bakteriellen Amplifikations- und/oder Selektionssequenzen ist, umfassend:
einen RNA-Polymerase I (polI)-Promotor und ein polI-Terminationssignal, eingefügt zwischen einem RNA-Polymerase II (polII)-Promotor und einem Polyadenylierungssignal;
am N- und/oder C-Terminus des Konstrukts eine Homopurin- oder Homopyrimidin-Peptid-Nukleinsäure (PNA)-Klammer-Bindesequenz, die ein stabiles PNA-DNA-PNA-Triplexhybrid mit zwei identischen PNA-Klammer-Halbstellen, die komplementär dazu sind, bildet, und optional eine PNA-Klammer-Sequenz, die jene zwei PNA-Klammer-Halbstellen umfasst, verbunden durch einen flexiblen Haarnadel-Linker, der drei 8-Amino-3,6-dioxaoctansäure-Einheiten enthält,
wobei eine PNA ein Nukleinsäureanalogon ist, bei dem das Desoxyribosephosphat-Rückgrat durch ein Polyamid-Rückgrat, das 2-Aminoethylglycin-Einheiten enthält, ausgetauscht wurde und wobei eine PNA-Klammer zwei identische PNA-Sequenzen umfasst, verbunden durch einen flexiblen Haarnadel-Linker; und
mindestens ein Influenzavirus-Gensegment, eingefügt zwischen dem polI-Promotor und dem polI-Terminationssignal.

2. Das lineare Expressionskonstrukt gemäß Anspruch 1, wobei das virale Segment vom Influenzvirus des Typs A, B oder C stammt.

3. Das lineare Expressionskonstrukt gemäß Anspruch 1, wobei das virale Gensegment ausgewählt ist aus der Gruppe bestehend aus einem PA-, PB1-, PB2-, HA-, NA-, NP-, M-, NS-Gensegment oder einem Teil davon vom Influenzvirus.

4. Das lineare Expressionskonstrukt gemäß einem der Anspräche 1 bis 3, wobei das virale NS-Gensegment ein unfunktionales NS1-Protein exprimiert.

5. Das lineare Expressionskonstrukt gemäß einem der Anspräche 1 bis 4, wobei das virale Gensegment eine cDNA-Kopie oder ein RT-PCR-Amplifikationsprodukt von jenem Segment ist.

6. Ein Satz von linearen Expressionskonstrukten, die mindestens zwei Expressionskonstrukte gemäß den Ansprüchen 1 bis 5 enthalten.

7. Ein Satz von acht linearen Expressionskonstrukten gemäß Anspruch 6, wobei jedes mindestens ein virales Gensegment von PA, PB1, PB2, HA, NA, NP, M und NS oder einen Teil davon vom Influenzavirus enthält.

8. Eine Wirtszelle, die mindestens ein lineares Expressionskonstrukt gemäß einem der Anspräche 1 bis 7 umfasst.

9. Ein Verfahren zur Herstellung eines linearen Expressionskonstrukts gemäß einem der Anspräche 1 bis 5, wobei:
ein erstes DNA-Fragment, das ein Influenzavirus-Gensegment enthält, das zwischen einer Sequenz, die homolog zum polI-Promotor ist, und einer Sequenz, die homolog zum polI-Terminator ist, eingefügt ist,
ein zweites DNA-Fragment, das in der folgenden Reihenfolge eine Schutzsequenz, eine poly-A-Signalsequenz, eine polI-Promotor-Sequenz und eine überlappende Sequenz von mindestens 12 Nukleotiden, die komplementär zum viralen Segment sind, enthält, und
ein drittes DNA-Fragment, das in der folgenden Reihenfolge eine Schutzsequenz, einen CMV-Promotor, eine polI-Terminator-Sequenz und eine überlappende Sequenz von mindestens 12 Nukleotiden, die komplementär zum viralen Segment sind, enthält,
mittels überlappender PCR miteinander fusioniert und aufgereinigt werden.

10. Ein Verfahren gemäß Anspruch 9, das mindestens zwei Aufreinigungsschritte umfasst.

## Revendications

1. Construction d'expression linéaire dépourvue de toute séquence d'amplification et/ou de sélection bactérienne à base de plasmide classique comprenant :
un promoteur d'ARN polymérase I (polI) et un signal de terminaison de poli, insérés entre un promoteur d'ARN polymérase II (polII) et un signal de polyadénylation ;
à l'extrémité N- et/ou C-terminale de la construction,
une séquence de liaison de pince d'acide nucléique peptidique (PNA) homopurique ou homopyrimidique qui forme un triplex PNA-ADN-PNA hybride avec deux demi-sites de pince de PNA identiques qui y sont complémentaires, et éventuellement une séquence de pince de PNA comprenant lesdits deux demi-sites de pince de PNA joints par un lieur en épingle à cheveux flexible contenant trois unités d'acide 8-amino-3,6-dioxaoctanoïque,
dans laquelle un PNA est un analogue d'acide nucléique dans lequel le squelette désoxyribose-phosphate a été échangé avec un squelette polyamide contenant des unités de 2-aminoéthyl-glycine, et dans laquelle une pince de PNA comprend deux séquences de PNA identiques jointes par un lieur en épingle à cheveux flexible ; et
au moins un segment de gène du virus de la grippe inséré entre le promoteur de polI et le signal de terminaison de polI.

2. Construction d'expression linéaire selon la revendication 1, dans laquelle le segment viral provient du virus de la grippe de type A, B ou C.

3. Construction d'expression linéaire selon la revendication 1, dans laquelle le segment de gène viral est choisi dans le groupe constitué d'un segment de gène PA, PB1, PB2, HA, NA, NP, M, NS ou de l'une de ses parties du virus de la grippe.

4. Construction d'expression linéaire selon l'une quelconque des revendications 1 à 3 dans laquelle le segment de gène NS viral exprime une protéine NS1 non fonctionnelle.

5. Construction d'expression linéaire selon l'une quelconque des revendications 1 à 4 dans laquelle le segment de gène viral est une copie d'ADNc ou un produit d'amplification par RT-PCR dudit segment.

6. Ensemble de constructions d'expression linéaires contenant au moins deux constructions d'expression selon les revendications 1 à 5.

7. Ensemble de huit constructions d'expression linéaires selon la revendication 6 chacune contenant au moins un segment de gène viral de PA, PB1, PB2, HA, NA, NP, M et NS ou l'une de ses parties du virus de la grippe.

8. Cellule hôte comprenant au moins une construction d'expression linéaire selon l'une quelconque des revendications 1 à 7.

9. Procédé de production d'une construction d'expression linéaire selon l'une quelconque des revendications 1 à 5 dans lequel :
un premier fragment d'ADN, contenant un segment de gène du virus de la grippe inséré entre une séquence homologue au promoteur de pol I et une séquence homologue au terminateur de pol I,
un deuxième fragment d'ADN, contenant dans l'ordre suivant une séquence de protection, une séquence signal poly A, une séquence promoteur de pol I, et une séquence chevauchante d'au moins 12 nucléotides complémentaire du segment viral et
un troisième fragment d'ADN, comprenant dans l'ordre suivant une séquence de protection, un promoteur du CMV, une séquence terminateur de pol I, et une séquence chevauchante d'au moins 12 nucléotides complémentaire du segment viral
sont fusionnés ensemble par l'intermédiaire d'une PCR chevauchante et purifiés.

10. Procédé selon la revendication 9 comprenant au moins deux étapes de purification.


<110> AVIR Green Hills Biotechnology Research Development Trade AG
<120> Novel constructs for expression of influenza virus
<130> PCRF/EP
<160> 6
<170> PatentIn version 3.3
<210> 1
   <211> 42
   <212> DNA
   <213> Influenza A virus
<400> 1
   cgaagttggg ggggagcaaa agcaggggat aattctatta ac 42
<210> 2
   <211> 44
   <212> DNA
   <213> Influenza A virus
<400> 2
   gccgccgggt tattagtaga aacaagggtg tttttaatta atgc 44
<210> 3
   <211> 32
   <212> DNA
   <213> Influenza A virus
<400> 3
   cctgcttttg ctccccccca acttcggagg tc 32
<210> 4
   <211> 34
   <212> DNA
   <213> artifical sequence
<400> 4
   ggggtatcag ggttattgtc tcatgagcgg atac 34
<210> 5
   <211> 36
   <212> DNA
   <213> Influenza A virus
<400> 5
   ccttgtttct actaataacc cggcggccca aaatgc 36
<210> 6
   <211> 32
   <212> DNA
   <213> artificial sequence
<220>
   <223> plasmid sequence
<400> 6
   ccccttggcc gattcattaa tgcagctggt tc 32